# EUROPEAN PATENT APPLICATION

(11) **EP 3 093 287 A1**
(43) Date of publication of application: **16.11.2016**
(21) Application number: 15382252.3
(22) Date of filing: 15.05.2015
(51) Int. Cl.: C07D 295/26

(54) **AN IODINE CATALYSED PROCESS FOR PREPARING HETEROCYCLIC NITROGEN-CONTAINING COMPOUNDS**

(71) Applicant: Fundació Institut Català D'investigació Quimica, 43007 Tarragona (ES); Institució Catalana de Recerca i Estudis Avançats, 08010 Barcelona (ES)
(72) Inventor: Muñy Klein, Kilian, 43007 Tarragona (ES); Martínez, Claudio, 43005 Tarragona (ES)
(74) Representative: ZBM Patents - Zea, Barlocci & Markvardsen

(57) **Abstract**

The present invention relates to a process for preparing a 5- or 6-membered nitrogen-containing heterocyclic compound which comprises contacting a compound comprising a (C₄-C₅)alkylsulfonamide moiety, with a source of iodine and a metal-free oxidant; wherein: the compound comprising a (C₄-C₅)alkylsulfonamide moiety is capable of suffering an intramolecular cyclization converting a C-H bond into a C-N bond, in order to form a 5- or 6-membered nitrogen-containing heterocyclic ring; the amount of the iodine source is comprised from 0.5 mol% to 50 mol% of the moles of the compound comprising a (C₄-C₅)alkylsulfonamide moiety; and the amount of metal-free oxidant is comprised from 1 to 5 equivalents of the compound comprising a (C₄-C₅)alkylsulfonamide moiety; and the process is carried out at a temperature comprised from 0°C to 50°C in the presence of an organic solvent and under exposure to a source of light; a compound of formula I(OCOR₁₁); its use as a catalyst upon light irradiation; and a compound of formula [I(OCOR₁₁)₂]M.

## Description

The present invention relates to a process for preparing heterocyclic nitrogen-containing compounds. Particularly, to a process for preparing 5- or 6-membered nitrogen-containing heterocyclic compounds, which comprises contacting a compound comprising a (C₄-C₅)alkylsulfonamide moiety with a iodine source and a metal-free oxidant. It also relates to a compound of formula I(OCOR₁₁) and a compound of formula I[(OCOR₁₁)₂M; and the use of a compound of formula I(OCOR₁₁) as a catalyst upon light irradiation.

### BACKGROUND ART

Nitrogen-halogen bonds have a long history in the synthesis of pyrrolidines and related heterocyclic structures through the amination reaction of a distant carbon-hydrogen bond. For such approaches with preformed chlorinated and brominated amines, the transformation is known as Hofmann-Löffler reaction. Despite the great attractiveness of such an approach, harsh conditions have prevented wider applications.

Modifications of the above mentioned process have been developed in the state of the art. One of these approaches include the *in situ* formation of the corresponding N-iodinated amides from the combined use of at least equimolar amounts of an iodine source such as molecular iodine and a large excess of commonly available iodine(III) reagents (hypervalent iodine(III) reagents) with the requirement of exposure to a source of external light.

These reactions usually start from electron-acceptor substituted nitrogen groups in the presence of a transition-metal catalyst to promote the reaction. For example, PhI(OAc)₂ has been used as an oxidizing reagent in the presence of Rh- or Ru-catalyst for the C-H amination in the synthesis of 5- or 6- membered ring heterocycles. In these procedures, a primary amide is oxidized with PhI(OAc)₂ to an imidoiodane, which is in turn decomposed by a metal complex to generate the corresponding metallonitrene. The following cyclization occurs via C-H insertion to give the heterocycles.

A modification of the above mentioned process under metal-free conditions for intramolecular cyclization of compounds comprising a sulfonamide moiety has been developed. This process has been used for the preparation of 5-membered heterocyclic compounds by using stoichiometric amounts of the iodine source and over-stoichiometric amounts of the oxidant reagent. The reaction is carried out by reacting a compound comprising a sulfonamide moiety with an equivalent of molecular iodine and and excess of a metal-free oxidant (i.e. 3 equivalents of PhI(OAc)₂). However, this cyclization is very sensitive to the reaction conditions such as temperature, solvent and amount of the iodine source and oxidant. (Renhua Fan et al., "δ and α SP3 C-H bond oxidation of sulfonamides with PhI(OAc)2/I2 under Metal-free conditions", J.Org.Chemistry, 2007, vol. 72, pp. 8994-8997).

Unfortunately, these processes still have certain limitations that preclude their industrial application. For example, the use of metal catalysts or the introduction of metal additives, as well as the low solubility of oxidant reagents in the organic solvents and the need for excess of reagents are among these limitations.

Therefore, from what is known in the art it is derived that there is still need of providing metal-free catalysts (under-stoichiometric amount) for the preparation of a wide range of 5- or 6- membered heterocyclic ring compounds having high reactivity and/or diastereoselectivity under mild conditions.

### SUMMARY OF THE INVENTION

Inventors have found a highly effective catalyst mixture useful for the preparation of a wide range of 5- or 6- membered heterocyclic ring compounds under mild conditions and under exposure to a source of light. The mixture has an under-stoichiometric amount of an iodine source and a metal-free oxidant in stoichiometric amount with a specific molar ratio among them. The catalyst mixture of the present invention allows the formation of the active specie of formula I(OCOR₁₁) as defined above, which is specially advantageous because it avoids the use of metals, as well as the use of high amounts of the iodine source and the oxidant. Further, the use of the mixture of the invention provides a cleaner reaction outcoming in the oxidation of a wide range of compounds comprising a (C₄-C₅)alkylsulfonamide moiety without forming undesirable by-products.

Thus, a first aspect of the invention relates to a process for preparing a 5- or 6-membered nitrogen-containing heterocyclic compound which comprises contacting a compound comprising a (C₄-C₅)alkylsulfonamide moiety, with a source of iodine and a metal-free oxidant; wherein: the compound comprising a (C₄-C₅)alkylsulfonamide moiety is capable of suffering an intramolecular cyclization converting a C-H bond into a C-N bond, in order to form a 5- or 6-membered nitrogen-containing heterocyclic ring; the amount of the iodine source is comprised from 0.5 mol% to 50 mol% of the moles of the compound comprising a (C₄-C₅)alkylsulfonamide moiety; and the amount of metal-free oxidant is comprised from 1 to 5 equivalents of the compound comprising a (C₄-C₅)alkylsulfonamide moiety; and the process is carried out at a temperature comprised from 0°C to 50°C in the presence of an organic solvent and under exposure to a source of light.

A second aspect of the invention relates to a compound of formula I(OCOR₁₁), wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group consisting of fluoro, chloro and bromo.

The third aspect of the invention relates to the use of the compound as defined in the second aspect of the invention, as a catalyst to be used under exposure to a source of light.

Finally, the fourth aspect of the invention relates to a compound of formula I[(OCOR₁₁)₂]M, wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group fluoro, chloro and bromo; and M is a cation selected from the group consisting of H⁺, a cation of an alkaline metal and a cation of a compound of formula N(R₁₂)₄ wherein each R₁₂ is independently selected from the group consisting of hydrogen and (C₁-C₆)alkyl.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

In the context of the invention, the term "catalyst" or "catalytically effective amount" or "under-stoichiometric amount" refers to the fact that the amount of iodine source is much smaller than the amount of the compound comprising the (C₄-C₅)alkylsulfonamide. The amount is expressed as mol% of the iodine source used to prepare the active catalytic compound with the metal-free oxidant in relation to the compound comprising a (C₄-C₅)alkylsulfonamide moiety.

The term "alkyl" refers to a saturated straight, or branched hydrocarbon chain which contains the number of carbon atoms specified in the description or claims. Examples include the group methyl, ethyl, propyl, isopropyl, butyl, isobutyl, sec-butyl, *tert*-butyl, n-pentyl, and n-hexyl.

The term "monocyclic ring" refers to a 5- to 6- membered ring or 5- to 6-membered ring bridged or fused with one or more 5- to 12- membered rings. The rings can be saturated, partially unsaturated or aromatic, where the members of the rings are independently selected from C, CH, CH₂, O, N, NH, and S; being one or more of the hydrogen atoms of the members optionally substituted by a radical selected from the group consisting of (C₁-C₆)alkyl, (C₁-C₆)haloalkyl, halogen, (C₁-C₆)alkyl-O-, nitro, cyano, (C₁-C₆)alkyl-CO-,(C₁-C₆)alkyl-SO₂-, *p*-tolyl-SO₂- and (C₁-C₆)alkyl-O-CO-; Examples include, among others, phenyl, isoquinolyl, quinolyl, indenyl, triazolyl, tetrazolyl, indolyl, furyl, pyrrolyl, thienyl, benzofuryl, pyridyl, naphthyridyl, imidazolyl, benzoxazolyl, benzisoxazolyl, benzotriazolyl, biphenyl, 1-naphthyl, and 2-naphthyl.

The term "alkenyl" refers to a saturated straight, or branched alkyl chain which contains the number of carbon atoms specified in the description or claims and that also contains one or more double bonds. Examples include, among others, ethenyl, 1-propen-1-yl, 1-propen-2-yl, 3-propen-1-yl, 1-buten-1-yl, 1-buten-2-yl, 3-buten-1-yl, 3-buten-2-yl, 2-buten-1-yl, 2-buten-2-yl, 2-methyl-1-propen-1-yl, 2-methyl-2-propen-1-yl, 1,3-butadien-1-yl, 1,3-butadien-2-yl, and 2-hexenyl.

The term "alkynyl" refers to a saturated straight, or branched alkyl chain which contains the number of carbon atoms specified in the description or claims and that also contains one or more triple bonds. Examples include, among others, ethynyl, 1-propynyl, 2-butynyl, 1,3-butadinyl, 4-pentynyl, and 1-hexynyl.

The term "haloalkyl" refers to a group resulting from the replacement of one or more hydrogen atoms from an alkyl group with one or more halogen atoms, which can be the same or different. Examples include, among others, trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, and nonafluorobutyl.

The term "(C₆-C₂₀)aryl" as used herein, whether used alone or as part of another group, is defined as a radical derived from one of the known ring systems with 1-4 phenyl rings, having from 6 to 20 carbon atoms wherein each one of the rings forming said ring system is isolated or, partially or totally fused. Non-limitative examples include phenyl, naphthyl, phenantryl, anthracenyl, biphenylyl and phenylylnaphthalene.

The term "(C₅-C₂₀)heteroaryl" as used herein, whether used alone or as part of another group, is defined as a radical derived from one of the known ring systems with 1-4 5-membered or 6-membered aromatic rings, and having from 5 to 20 atoms, each member being independently selected from the group consisting of C, O, N, NH, S and CH, and wherein each one of the rings forming said ring system is isolated or, partially or totally fused. Non-limitative examples include pyrrolyl, furyl, thiophenyl, pyrazolyl, indolyl, benzofuryl, benzothiofuryl, quinolyl, isoquinolyl, naphtyridyl, imidazolyl, triazolyl, tetrazolyl, oxazolyl, isoxazolyl, thiazolyl, isothiazolyl, dithiolanyl, pyridyl, pyridazinyl, pyrimidinyl, and pyrazinyl.

As mentioned above, an aspect of the present invention refers to a process for preparing a wide range of 5- or 6- membered nitrogen-containing heterocyclic compound by using the mixture of a iodine source and metal-free oxidant, wherein the iodine source is in an under-stoichiometric amount and the amount of metal-free oxidant is comprised from 1 to 5 equivalents of the compound comprising a (C₄-C₅)alkylsulfonamide moiety. The process of the invention is carried out under exposure to a source of light and mild conditions.

In comparison with the processes of the state of the art, the use of under-stoichiometric amounts (i.e. catalytic amount) of the iodine source provides a cleaner reaction outcoming in the oxidation of a wide range of compounds comprising a (C₄-C₅)alkylsulfonamide moiety without forming undesirable by-products. Further, the process of the invention, which proceeds within an iodine catalysis manifold is operationally simple, and metal-free. It is advantageous, because it is an environmentally friendly process to prepare a wide range of 5- or 6- membered nitrogen-containing heterocyclic compounds.

In an embodiment of the invention, optionally in combination with one or more features of the various embodiments described above or below, the process is that wherein the compound comprising a (C₄-C₅)alkylsulfonamide moiety is a compound of formula (1), and the 5- or 6-membered nitrogen-containing heterocyclic compound is the corresponding 5- or 6- membered nitrogen containing heterocyclcic compound of formula (2): 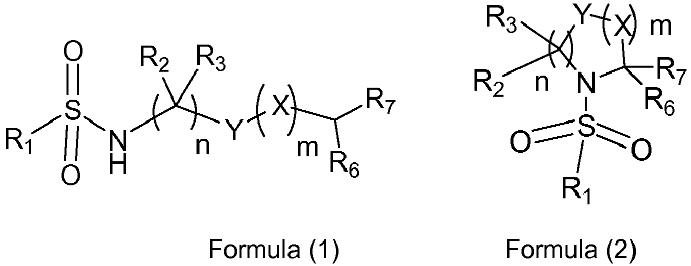 wherein: X is a heteroatom selected from the group consisting of NPG, O and S; Y is selected from the group consisting of CR₄R₅ and C=CR₄R₅; R₁ is selected from (C₁-C₅)alkyl and phenyl, being the groups alkyl and phenyl unsubstituted or substituted at any position with one or more radicals independently selected from the group consisting of (C₁-C₅)alkyl, nitro, ((C₁-C₄)alkyl)₃-Si-, halogen and hydroxy; R₂, R₃, R₄, R₅, R₆ and R₇ are independently selected from the group consisting of hydrogen, (C₁-C₆)alkyl, monocyclic ring, benzyl, (C₂-C₆)alkenyl, (C₂-C₆)alkynyl, being the groups (C₁-C₆)alkyl, benzyl, (C₂-C₆)alkenyl and (C₂-C₆)alkynyl unsubstituted or substituted at any position with one or more radicals independently selected from the group consisting of halogen, hydroxy, N(R₁₂)₂ wherein each R₁₂ is independently selected from hydrogen and (C₁-C₆)alkyl, (C₁-C₆)alkyl-O-; or alternatively, R₂ and R₃; R₄ and R₅; R₆ and R₇; R₂ and R₄; R₂ and R₅; R₃ and R₄; and R₃ and R₅; in combination with the adjacent carbon atoms form a 5 to 6 membered ring, saturated or partially unsaturated, optionally bridged or fused to a 5 to 6 membered ring; the members of the rings being independently selected from C, CH, CH₂, O, N, NH, and S; being one or more of the hydrogen atoms of the members optionally substituted by a radical selected from the group consisting of (C₁-C₅)alkyl, nitro, halogen and hydroxy; PG is an amine protecting group selected from the group consisting of the groups of formula -(CO)OR₈; benzyl, and -SO₂R₉; R₈ is selected from the group consisting of (C₁-C₆)alkyl, benzyl and fluorenyl; R₉ is selected from (C₁-C₅)alkyl and phenyl, being the groups alkyl and phenyl unsubstituted or substituted at any position with one or more radicals independently selected from the group consisting of (C₁-C₅)alkyl, nitro, ((C₁-C₄)alkylh-Si-, halogen and hydroxy; n is an integer selected from 1-3; m is an integer selected from 0-1; and with the proviso that when m is 0 then n is 2 or 3, and when m is 1, then n is 1 or 2.

In an embodiment, optionally in combination with one or more features of the various embodiments described above or below, the process is that wherein m is 0 and n is 2; or alternatively m is 1 and n is 1. This is advantageous because the process allows the formation of a wide range of 5-membered heterocycle compound. In a particular embodiment, optionally in combination with one or more features of the various embodiments described above or below, the process is that where m is 0 and n is 2, then the process yields a wide range of unsubstituted or substituted pyrrolidine compounds.

In an embodiment, optionally in combination with one or more features of the various embodiments described above or below, the process is that wherein n is 1; and R₂ and R₃ are hydrogen.

In an embodiment, optionally in combination with one or more features of the various embodiments described above or below, the process is that wherein R₁ is unsubstituted or substituted phenyl. Preferably, the process is that wherein R₁ is a substituted phenyl as defined above. Particularly, in an embodiment, optionally in combination with one or more features of the various embodiments described above or below, the process is that wherein R₁ is selected from the group consisting of 4-methylphenyl- (tosyl group), 2-nitrophenyl- (nosyl group), phenyl, 2-4-dinitrophenyl and 4-nitrophenyl- (nosyl group). More particularly, in an embodiment, optionally in combination with one or more features of the various embodiments described above or below, the process is that wherein R₁ is selected from the group consisting of 4-methylphenyl-(tosyl group), 2-nitrophenyl- (nosyl group) and 4-nitrophenyl-(nosyl group).

In an alternative embodiment, optionally in combination with one or more features of the various embodiments described above or below, the process is that wherein R₁ is unsubstituted or substituted (C₁-C₅)alkyl as defined above. Particularly, in an embodiment, optionally in combination with one or more features of the various embodiments described above or below, the process is that wherein R₁ is selected from the group consisting of methyl-(mesyl group), *tert*-butyl and 2-(trimethylsilyl)ethyl- (SES group). More particularly, in an embodiment, optionally in combination with one or more features of the various embodiments described above or below, the process is that wherein R₁ is selected from the group consisting of methyl- (mesyl group) and 2-(trimethylsilyl)ethyl- (SES group).

In an embodiment, optionally in combination with one or more features of the various embodiments described above or below, the process is that wherein Y is CR₄R₅, and R₄ and R₅ are as defined in the present invention. In an embodiment, optionally in combination with one or more features of the various embodiments described above or below, the process is that wherein Y is CR₄R₅, and R₄ and R₅ are independently selected from the group consisting of hydrogen, (C₁-C₆)alkyl and monocyclic ring; particularly, Y is CR₄R₅, and R₄ and R₅ are independently selected from hydrogen, methyl, ethyl, phenyl and cyclohexyl.

In an embodiment, optionally in combination with one or more features of the various embodiments described above or below, the process is that wherein Y is C=CR₄R₅, and R₄ and R₅ are as defined in the present invention; preferably, Y is C=CR₄R₅, and R₄ and R₅ are hydrogen.

In an embodiment, optionally in combination with one or more features of the various embodiments described above or below, the process is that wherein R₆ and R₇ are independently selected from the group consisting of hydrogen, (C₁-C₆)alkyl, monocyclic ring and benzyl. Particularly, the process is that wherein R₆ and R₇ are independently selected from hydrogen, methyl, ethyl, phenyl, methoxy, 4-halo-phenyl, 4-methyl-phenyl, and 4-methoxy-phenyl.

In an embodiment, optionally in combination with one or more features of the various embodiments described above or below, the process is that wherein R₂ and R₄; R₂ and R₅; R₃ and R₄; and R₃ and R₅ in combination with the adjacent carbon atoms form a 5 to 6 membered ring, saturated or partially unsaturated, optionally bridged or fused to a 5 to 6 membered ring; the members of the rings being independently selected from C, CH, CH₂, O, N, NH, and S; being one or more of the hydrogen atoms of the members optionally substituted by a radical selected from the group consisting of (C₁-C₆)alkyl, (C₁-C₆)haloalkyl, halogen, (C₁-C₆)alkyl-O-, (C₁-C₆)alkyl-CO-, (C1-C6)alkyl-O-CO-, (C₁-C₆)alkyl-SO₂-, *p*-tolyl-SO₂-, nitro and cyano. In a particular embodiment, optionally in combination with one or more features of the various embodiments described above or below, the process is that wherein R₂ and R₄; R₂ and R₅; R₃ and R₄; and R₃ and R₅ in combination with the adjacent carbon atoms form a 5 to 6 membered ring, saturated or partially unsaturated, optionally bridged or fused to a 5 to 6 membered ring; the members of the rings being independently selected from C, CH, and CH₂.

In a particular embodiment, optionally in combination with one or more features of the various embodiments described above or below, the process is that wherein the compound comprising a (C₄-C₅)alkylsulfonamide moiety of formula 1 is one of the compound listed below which render the corresponding 5- or 6- membered nitrogen-containing heterocyclic compound listed below. For example the compound of formula (1 a) renders the corresponding compound of formula (2a).

In this particular embodiment, the compound of formula (1) is selected from the group consisting of:

| **Molecular formula** | | **Name** |
|---|---|---|
| 1a | 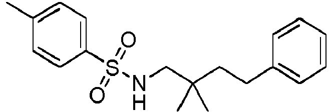 | *N*-(2,2-Dimethyl-4-phenylbutyl)-4-methylbenzenesulfonamide |
| 1b | 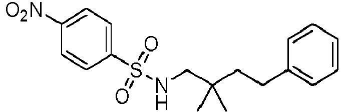 | *N*-(2,2-Dimethyl-4-phenylbutyl)-4-nitrobenzenesulfonamide |
| 1c | 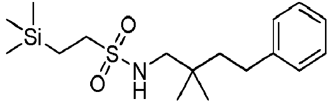 | *N*-(2,2-Dimethyl-4-phenylbutyl)-2-(trimethylsilyl)ethanesulfonamide |
| 1d | 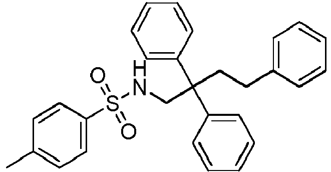 | 4-Methyl-N-(2,2,4-triphenylbutyl)benzenesulfonamide |
| 1e | 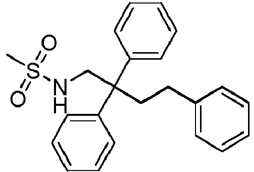 | *N*-(2,2,4-Triphenylbutyl)methanesulfonamide |
| 1f | 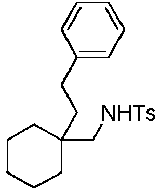 | 4-Methyl-*N*-((1-phenethylcyclohexyl)methyl)benzen esulfonamide |
| 1g | 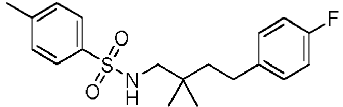 | N-(4-(4-Fluorophenyl)-2,2-dimethylbutyl)-4-methylbenzenesulfonamide |
| 1h | 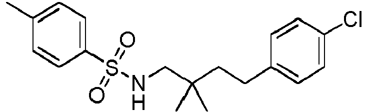 | *N*-(4-(4-Chlorophenyl)-2,2-dimethylbutyl)-4-methylbenzenesulfonamide |
| 1i | 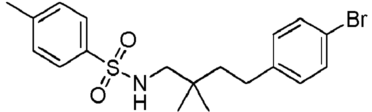 | *N*-(4-(4-Bromophenyl)-2,2-dimethylbutyl)-4-methylbenzenesulfonamide |
| 1j | 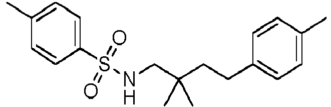 | *N*-(2,2-Dimethyl-4-(*p*-tolyl)butyl)-4-methylbenzenesulfonamide |
| 1k | 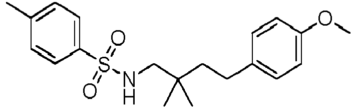 | *N*-(4-(4-Methoxyphenyl)-2,2-dimethylbutyl)-4-methylbenzenesulfonamide |
| 1l | 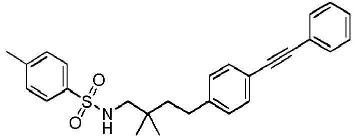 | *N*-(2,2-Dimethyl-4-(4-(phenylethynyl)phenyl)butyl)-4-methylbenzenesulfonamide |
| 1m | 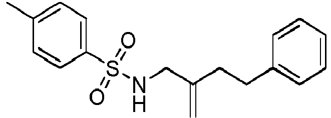 | 4-Methyl-*N*-(2-methylene-4-phenylbutyl)benzenesulfonamide |
| 1n-d1 | 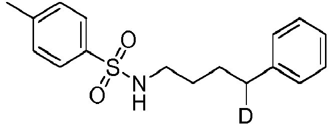 | 4-Methyl-*N*-(4-deutero,4-phenylbutyl)benzenesulfonamide |
| 1 n-d2 | 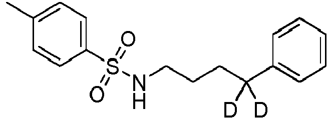 | 4-Methyl-*N*-(4,4-bisdeutero,4-phenylbutyl)benzenesulfonamide |
| 1o | 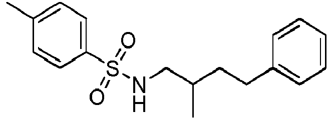 | 4-Methyl-*N*-(2-methyl-4-phenylbutyl)benzenesulfonamide |
| 1p | 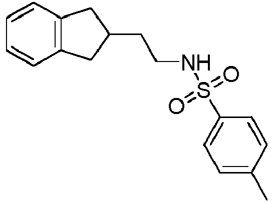 | *N*-(2-(2,3-Dihydro-1*H*-inden-2-yl)ethyl)-4-methylbenzenesulfonamide |
| 1q | 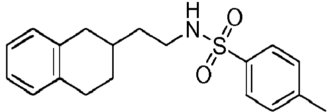 | 4-Methyl-*N*-(2-(1,2,3,4-tetrahydronaphthalen-2-yl)ethyl)benzenesulfonamide |
| 1r | 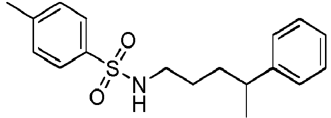 | 4-Methyl-*N*-(4-phenylpentyl)benzenesulfonamide |
| 1s | 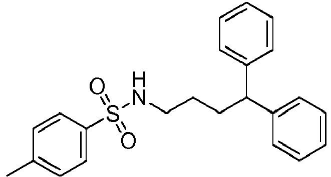 | *N*-(4,4-Diphenylbutyl)-4-methylbenzenesulfonamide |
| 1t | 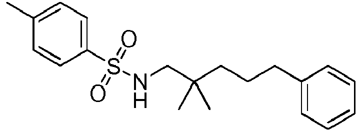 | *N*-(2,2-Dimethyl-5-phenylpentyl)-4-methylbenzenesulfonamide |
| 1u | 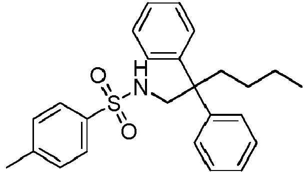 | *N*-(2,2-Diphenylhexyl)-4-methylbenzenesulfonamide |
| 1v | 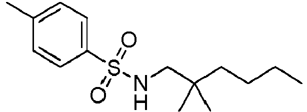 | *N*-(2,2-Dimethylhexyl)-4-methylbenzenesulfonamide |
| 1w | 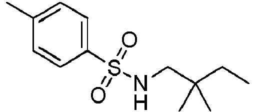 | *N*-(2,2-Dimethylbutyl)-4-methylbenzenesulfonamide |
| 1x | 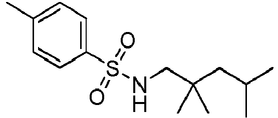 | 4-Methyl-*N*-(2,2,4-trimethylpentyl)benzenesulfonamide |
| 1y | 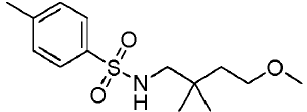 | *N*-(4-Methoxy-2,2-dimethylbutyl)-4-methylbenzenesulfonamide |
| 1z | 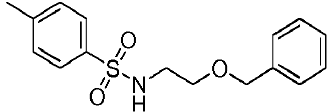 | *N*-(2-(Benzyloxy)ethyl)-4-methylbenzenesulfonamide |
| 1aa | 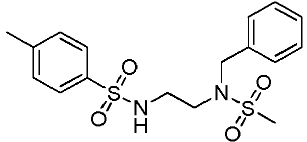 | *N-*(2-(N-Benzylmethylsulfonamido)ethyl)-4-methylbenzenesulfonamide |
| 1ab | 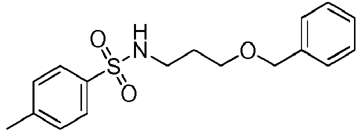 | *N*-(3-(Benzyloxy)propyl)-4-methylbenzenesulfonamide |
| 1ac | 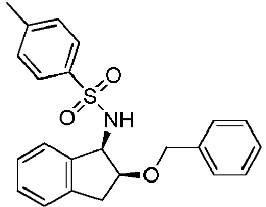 | *N*-((1*R*,2*S*)-2-(Benzyloxy)-2,3-dihydro-1H-inden-1-yl)-4-methylbenzenesulfonamide |
| 1ad | 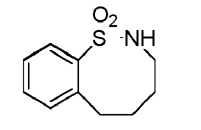 | 3,4,5,6-tetrahydro-2H-benzo[g][1,2]thiazocine 1,1-dioxide |
| 1ae | 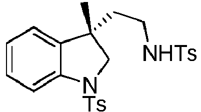 | 4-methyl-N-(2-(3-methyl-1-tosylindolin-3-yl)ethyl)benzenesulfonamide |

In this particular embodiment, the compound of formula (2) is selected from the group consisting of:

| **Molecular formula** | | **Name** |
|---|---|---|
| 2a | 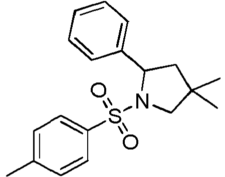 | 4,4-Dimethyl-1-((4-methylphenyl)sulfonyl)-2-phenylpyrrolidine |
| 2b | 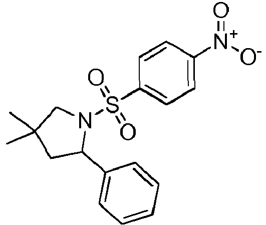 | 4,4-Dimethyl-1-((4-nitrophenyl)sulfonyl)-2-phenylpyrrolidine |
| 2c | 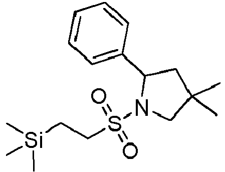 | 4,4-Dimethyl-2-phenyl-1-((2-(trimethylsilyl)ethyl)sulfonyl)pyrrolidine |
| 2d | 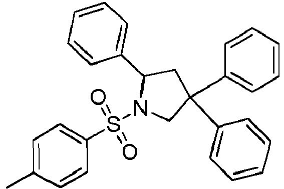 | 2,4,4-Triphenyl-1-tosylpyrrolidine |
| 2e | 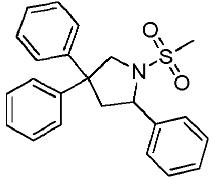 | 1-(Methylsulfonyl)-2,4,4-triphenylpyrrolidine |
| 2f | 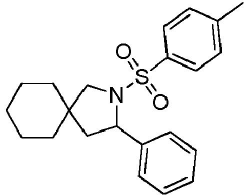 | 3-Phenyl-2-tosyl-2-azaspiro[4.5]decane |
| 2g | 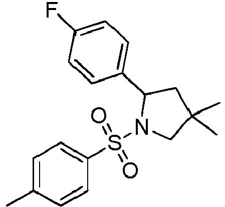 | 2-(4-Fluorophenyl)-4,4-dimethyl-1-tosylpyrrolidine |
| 2h | 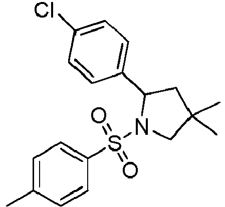 | 2-(4-Chlorophenyl)-4,4-dimethyl-1-tosylpyrrolidine |
| 2i | 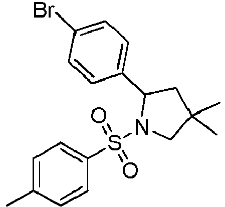 | 2-(4-Bromophenyl)-4,4-dimethyl-1-tosylpyrrolidine |
| 2j | 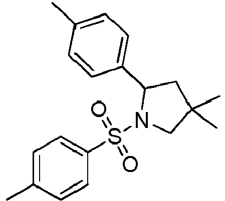 | 4,4-Dimethyl-2-(*p*-tolyl)-1-tosylpyrrolidine |
| 2k | 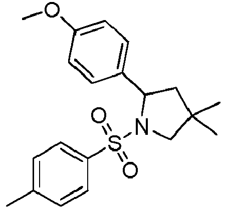 | 2-(4-Methoxyphenyl)-4,4-dimethyl-1-tosylpyrrolidine |
| 2l | 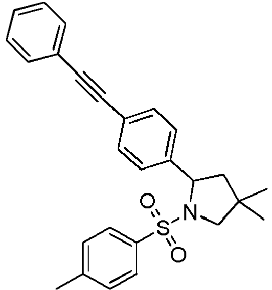 | 4,4-Dimethyl-2-(4-(phenylethynyl)phenyl)-1-tosylpyrrolidine |
| 2m | 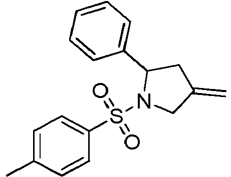 | 4-Methylene-2-phenyl-1-tosylpyrrolidine |
| 2n | 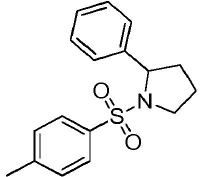 | 2-Phenyl-1-tosylpyrrolidine |
| 2o | 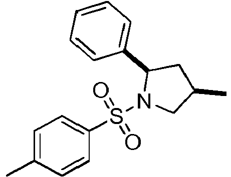 | (±)-*unlike*-4-Methyl-2-phenyl-1-tosylpyrrolidine |
| 2o' | 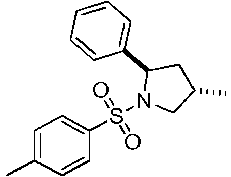 | (±)-*like*-4-Methyl-2-phenyl-1-tosylpyrrolidine |
| 2p | 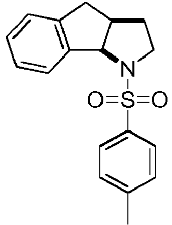 | (±)-*unlike*-1-Tosyl-1,2,3,3a,4,8b-hexahydroindeno[1,2-*b*]pyrrole |
| 2q | 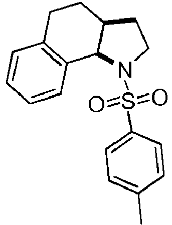 | 1-Tosyl-2,3,3a,4,5,9b-hexahydro-1 *H-*benzo[*g*]indole |
| 2r | 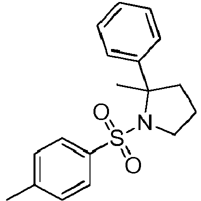 | 2-Methyl-2-phenyl-1-tosylpyrrolidine |
| 2s | 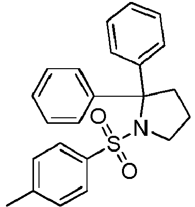 | 2,2-Diphenyl-1-tosylpyrrolidine |
| 2t | 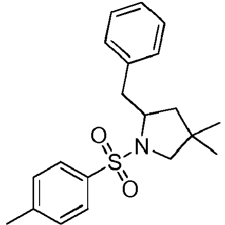 | 2-Benzyl-4,4-dimethyl-1-tosylpyrrolidine |
| 2u | 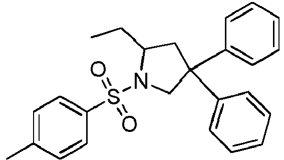 | 2-Ethyl-4,4-diphenyl-1-tosylpyrrolidine |
| 2v | 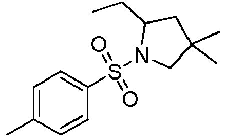 | 2-Ethyl-4,4-dimethyl-1-tosylpyrrolidine |
| 2w | 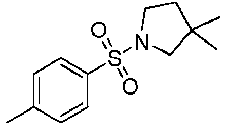 | 3,3-Dimethyl-1-tosylpyrrolidine |
| 2x | 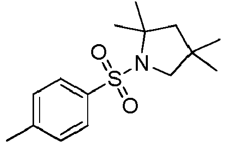 | 2,2,4,4-Tetramethyl-1-tosylpyrrolidine |
| 2y | 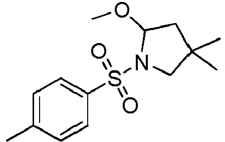 | 2-Methoxy-4,4-dimethyl-1-tosylpyrrolidine |
| 2z | 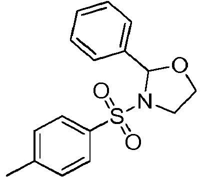 | 2-Phenyl-3-tosyloxazolidine |
| 2aa | 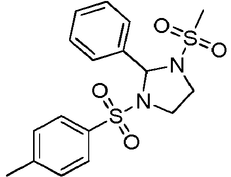 | 1-(Methylsulfonyl)-2-phenyl-3-tosylimidazolidine |
| 2ab | 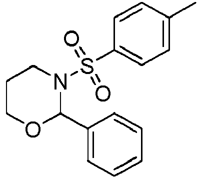 | 2-Phenyl-3-tosyl-1,3-oxazinane |
| 2ac | 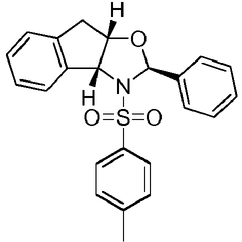 | (±)-(2*R*,3a*S*,8a*R*/2*S*,3a*R*,8a*S*)-2-Phenyl-3-tosyl-3,3a,8,8a-tetrahydro-2*H*indeno[ 1,2-*d*]oxazole |
| 2ad | 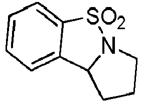 | 1,2,3,9b-tetrahydrobenzo[d]pyrrolo[1,2-b]isothiazole 5,5-dioxide |
| 2ae | 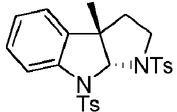 | 4-methyl-N-(2-(3-methyl-1-tosylindolin-3-yl)ethyl)benzenesulfonamide |

Without being bound to any theory, it is believed that the process for the preparation 5- or 6-membered nitrogen-containing heterocyclic compound of the present invention comprises an unique iodine-catalyzed oxidative amination of hydrycarbons that proceeds within two intertwined catalytic cycles. The process combines a radical chain reaction with an iodine catalysis that proceeds with a second iodine catalysis manifold.

For example, when the metal-free oxidant is a hypervalent iodine(III) reagent such as for example PhI(*m*CBA)₂, where *m*CBA means 3-chlorobenzoate, the initial reaction between molecular iodine and the hypervalent iodine(III) reagent PhI(mCBA)₂ provides formation of I(mCBA), which is the active catalyst. With every turnover, the reaction generates two equivalents of carboxylic acid. In fact, it seems that the active catalyst I(mCBA) might receive beneficial stabilisation through acid adduct formation. The process is summarized in the following scheme:

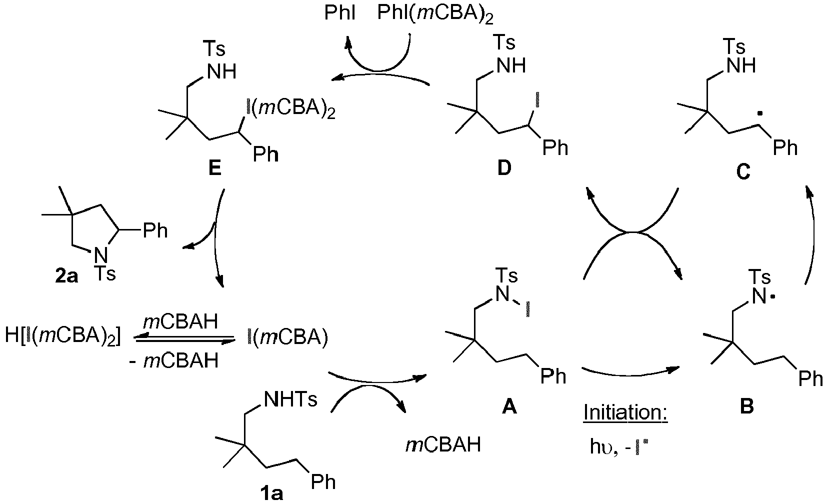

In an embodiment, optionally in combination with one or more features of the various embodiments described above or below, the process is that wherein the source of iodine is selected from molecular iodine (I₂), N-iodosuccinimide, and salts of formula MI, wherein M is a cation selected from the group consisting of H⁺, a cation of an alkaline metal and a cation of a compound of formula N(R₁₂)₄, being R₁₂ selected from the group consisting of hydrogen and (C₁-C₆)alkyl. In a preferred embodiment, optionally in combination with one or more features of the various embodiments described above or below, the process is that wherein the source of iodine is selected from the group consisting of molecular iodine (I₂), Nal, N-iodosuccinimide, N(CH₃)₄I and KI. In a more preferred embodiment, optionally in combination with one or more features of the various embodiments described above or below, the process is that wherein the source of iodine is selected from molecular iodine (I₂) and KI; even more preferably molecular iodine (I₂).

In an embodiment, optionally in combination with one or more features of the various embodiments described above or below, the process is that wherein the metal-free oxidant is selected from the group consisting of a bleach, a peroxide, a peracid, an hypervalent iodine(III) reagent, and a persulfate. Examples of peroxides suitable for the present invention include hydrogen peroxide, *tert*-butylhydroperoxide (TBHP) and cumene hydroxyperoxide (CHP). Examples of persulfates suitable for the present invention include alkaline metal salts of peroxymonosulfuric acid (oxone).

In an embodiment, optionally in combination with one or more features of the various embodiments described above or below, the process is that wherein the metal-free oxidant is selected from a peracid and an hypervalent iodine(III) reagent.

In an embodiment of the invention, optionally in combination with one or more features of the various embodiments described above or below, the process is that wherein the hypervalent iodine (III) reagent is a compound of formula (3):

(C₆-C₂₀)aryl-I-(OR)(OR') Formula (3)

wherein: R and R' are independently selected from the group consisting of (C₁-C₆)alkyl, benzyl, (C₆-C₂₀)aryl, (C₅-C₂₀)heteroaryl; (C₆-C₂₀)aryl wherein one or more of the hydrogen atoms is replaced by a group selected from the group consisting of (C₁-C₆)alkyl, (C₁-C₆)alkyl-O-, (C₁-C₆)haloalkyl, nitro, cyano, (C₁-C₆)alkyl-CO-, (C₁-C₆)alkyl-CO-O, (C₁-C₆)alkyl-O-CO, hydroxy and halogen; and (C₅-C₂₀)heteroaryl wherein one or more of the hydrogen atoms is replaced by a group selected from the group consisting of (C₁-C₆)alkyl, (C₁-C₆)alkyl-O-, (C₁-C₆)haloalkyl, nitro, cyano, (C₁-C₆)alkyl-CO-, (C₁-C₆)alkyl-CO-O-, (C₁-C₆)alkyl-O-CO-, hydroxy and halogen.

In an embodiment, optionally in combination with one or more features of the various embodiments described above or below, the process is that wherein the metal-free oxidant is a hypervalent iodine(III) reagent of formula PhI(Z)₂, wherein Z is selected from -OCO(C₁-C₆)alkyl and -OCOR₁₀; and R₁₀ is phenyl; being the groups alkyl or phenyl unsubstituted or substituted at any position with one or more radicals independently selected from the group consisting of (C₁-C₅)alkyl, (C₁-C₅)alkyl-O-,halogen and hydroxy. Preferably, Z is selected from -OCO(C₂-C₆)alkyl and -OCOR₁₀, wherein R₁₀ is phenyl, being the alkyl or phenyl group substituted at any position with one or more halogen; Preferably, the metal-free oxidant is a hypervalent iodine(III) reagent of formula PhI(OCOC(CH₃)₃)₂ and PhI(3-chlorobenzoate)₂ (PhI(mCBA)₂ being mCBA 3-chlorobenzoate).

In an alternative embodiment, optionally in combination with one or more features of the various embodiments described above or below, the process is that wherein the metal-free oxidant is a peracid; particularly selected from the group consisting of 3-chloroperbenzoic acid (meta-chloroperbenzoic acid; mCPBA), and peracetic acid; preferably the metal-free oxidant is meta-chloroperbenzoic acid.

The process of the invention is carried out in the presence of an under-stoichoimetric amount of iodine source comprised from 0.5 mol% to 50 mol% of the moles of the compound comprising a (C₄-C₅)alkylsulfonamide moiety. It is advantageous because the iodine source is used in a catalytically effective amount and then, the reaction is cleaner and easier to purify because it generates less by-products. In an embodiment, optionally in combination with one or more features of the various embodiments described above or below, the process is that wherein the amount of iodine source is comprised from 0.5 mol% to 20 mol%; preferably comprised from 0.5 mol% to 10 mol%; more preferably comprised from 0.5 mol% to 1 mol%.

In an alternative embodiment, optionally in combination with one or more features of the various embodiments described above or below, the process is that wherein when the metal-free oxidant is a hypervalent iodine(III) reagent of formula PhI(OCO(C₁-C₆)alkyl)₂; preferably PhI(OCO(C₂-C₆)alkyl)₂ as defined above, then the amount of iodine source is comprised from 20 mol% to 50 mol% of the moles of the compound comprising a (C₄-C₅)alkylsulfonamide moiety; preferably 20 mol% to 30 mol%. In a particular embodiment, the amount of iodine source is comprised from 20 mol% to 50 mol% of the moles of the compound comprising a (C₄-C₅)alkylsulfonamide moiety; preferably from 20 mol% to 30 mol% and the metal-free oxidant is a hypervalent iodine(III) reagent of formula PhI(OCOC(CH₃)₃)₂ and PhI(OCOCH₃)₂.

In an alternative embodiment, optionally in combination with one or more features of the various embodiments described above or below, the process is that wherein when the metal-free oxidant is a hypervalent iodine(III) reagent of formula PhI(OCOR₁₀)₂ as defined above, then the amount of iodine source is comprised from 0.5 mol% to 20 mol%; of the moles of the compound comprising a (C₄-C₅)alkylsulfonamide moiety; preferably 0.5 mol% to 5 mol%. In a particular embodiment, the amount of iodine source is comprised from 0.5 mol% to 20 mol% of the moles of the compound comprising a (C₄-C₅)alkylsulfonamide moiety; preferably from 0.5 mol% to 1 mol%; and the metal-free oxidant is a hypervalent iodine(III) reagent of formula PhI(mCBA)₂ being mCBA 3-chlorobenzoate.

In an alternative embodiment, optionally in combination with one or more features of the various embodiments described above or below, the process is that wherein when the metal-free oxidant is a peracid as defined above, then the amount of iodine source is comprised from 2 mol% to 25 mol% of the moles of the compound comprising a (C₄-C₅)alkylsulfonamide moiety; preferably 10 mol% to 20 mol%. In a particular embodiment, the amount of iodine source is of 15 mol% of the moles of the compound comprising a (C₄-C₅)alkylsulfonamide moiety; and the metal-free oxidant is a peracid selected from the group consisting of meta-chloroperbenzoic acid (mCPBA) and peracetic acid.

The process of the invention is carried out in the presence of an amount of the metal-free oxidant comprised from 1 to 5 equivalents of the compound comprising a (C₄-C₅)alkylsulfonamide moiety. In an embodiment, optionally in combination with one or more features of the various embodiments described above or below, the process is that wherein the amount of the metal-free oxidant is comprised from 1 to 3 equivalents of the compound comprising a (C₄-C₅)alkylsulfonamide moiety; preferably the amount of the metal-free oxidant is 1 equivalent. It is advantageous, because the process of the invention can be carried out with only a single equivalent of metal-free oxidant, it means that the process does not require excess of oxidant, in contrast with the processes of the state of the art.

In an alternative embodiment, optionally in combination with one or more features of the various embodiments described above or below, the process is that wherein the metal-free oxidant is a hypervalent iodine(III) reagent of formula PhI(OCO(C₁-C₆)alkyl)₂; preferably PhI(OCO(C₂-C₆)alkyl)₂; and the amount of the metal-free oxidant is comprised from 1 to 3 equivalents of the compound comprising a (C₄-C₅)alkylsulfonamide moiety, preferably 1 equivalent; and the amount of iodine source is comprised 20 mol% to 50 mol% of the moles of the compound comprising a (C₄-C₅)alkylsulfonamide moiety; preferably 20 mol% to 30 mol%.

In an alternative embodiment, optionally in combination with one or more features of the various embodiments described above or below, the process is that wherein the metal-free oxidant is a hypervalent iodine(III) reagent of formula PhI(OCOR₁₀)₂ as defined above; preferably PhI(mCBA)₂ being mCBA 3-chlorobenzoate; and the amount of the metal-free oxidant is comprised from 1 to 3 equivalents of the compound comprising a (C₄-C₅)alkylsulfonamide moiety, preferably 1 equivalent; and the amount of iodine source is comprised from 0.5 mol% to 20 mol% of the moles of the compound comprising a (C₄-C₅)alkylsulfonamide moiety; preferably 0.5 mol% to 1 mol%.

In an alternative embodiment, optionally in combination with one or more features of the various embodiments described above or below, the process is that wherein the metal-free oxidant is a peracid as defined above, preferably selected from meta-chloroperbenzoic acid (mCPBA), and peracetic acid; and the amount of the metal-free oxidant is comprised from 1 to 3 equivalents of the compound comprising a (C₄-C₅)alkylsulfonamide moiety, preferably 1 equivalent; and the amount of iodine source is comprised from 10 mol% to 25 mol% of the moles of the compound comprising a (C₄-C₅)alkylsulfonamide moiety; preferably the amount of iodine source is of 15 mol% of the moles of the compound comprising a (C₄-C₅)alkylsulfonamide moiety.

In a preferred embodiment, optionally in combination with one or more features of the various embodiments described above or below, the process is that wherein the metal-free oxidant is meta-chloroperbenzoic acid (mCPBA) and the amount of the metal-free oxidant is comprised from 1 to 3 equivalents of the compound comprising a (C₄-C₅)alkylsulfonamide moiety, preferably 1 equivalent; and the amount of iodine source is comprised from 10 mol% to 25 mol% of the moles of the compound comprising a (C₄-C₅)alkylsulfonamide moiety; preferably the amount of iodine source is of 15 mol% of the moles of the compound comprising a (C₄-C₅)alkylsulfonamide moiety.

Some embodiments of the invention are specially advantageous because the process is carried out in the presence of a catalytically effective amount of the iodine source and without an excess of the metal-free oxidant. It means that the process of the invention is more economical, cleaner and more environmentally-friendly than the processes disclosed in the state of the art.

The process of the invention is carried out in the presence of an organic solvent. In an embodiment, optionally in combination with one or more features of the various embodiments described above or below, the process is that wherein the metal-free oxidant is a hypervalent iodine(III) reagent as defined above, then the solvent is a halogenated solvent; more particularly a chlorinated solvent; and preferably a chlorinated solvent selected from the group consisting of CH₂Cl₂, (CH₂Cl)₂, CHCl₃, Cl₃CCH₂, Cl₂C=CHCl, (CHCl₂)₂ and CCl₄; more preferably (CH₂Cl)₂.

In an alternative embodiment, optionally in combination with one or more features of the various embodiments described above or below, the process is that wherein the metal-free oxidant is a hypervalent iodine(III) reagent of formula PhI(OCO(C₁-C₆)alkyl)₂; preferably PhI(OCO(C₂-C₆)alkyl)₂; and the amount of the metal-free oxidant is comprised from 1 to 3 equivalents of the compound comprising a (C₄-C₅)alkylsulfonamide moiety, preferably 1 equivalent; the amount of iodine source is comprised 20 mol% to 50 mol% of the moles of the compound comprising a (C₄-C₅)alkylsulfonamide moiety; preferably 20 mol% to 30 mol%; and the process is carried out in the presence of dichloroethane.

In an alternative embodiment, optionally in combination with one or more features of the various embodiments described above or below, the process is that wherein the metal-free oxidant is a hypervalent iodine(III) reagent of formula PhI(OCOR₁₀)₂ as defined above; preferably PhI(mCBA)₂ being mCBA 3-chlorobenzoate; and the amount of the metal-free oxidant is comprised from 1 to 3 equivalents of the compound comprising a (C₄-C₅)alkylsulfonamide moiety, preferably 1 equivalent; the amount of iodine source is comprised from 0.5 mol% to 20 mol% of the moles of the compound comprising a (C₄-C₅)alkylsulfonamide moiety; preferably 0.5 mol% to 1 mol%; and the process is carried out in the presence of dichloroethane.

In an embodiment, optionally in combination with one or more features of the various embodiments described above or below, the process is that wherein when the metal-free oxidant is a peracid as defined above, then the solvent is selected from acetonitrile, benzonitrile, acetic acid, methanol, trifluoroethane, ethylacetate, dichloroethane, and mixtures of acetonitrile/*tert*-butanol and dichlorethane/*tert*-butanol; preferably a mixture of acetonitrile/*tert*-butanol.

In an alternative embodiment, optionally in combination with one or more features of the various embodiments described above or below, the process is that wherein the metal-free oxidant is a peracid as defined above, preferably selected from meta-chloroperbenzoic acid (mCPBA), and peracetic acid; and the amount of the metal-free oxidant is comprised from 1 to 3 equivalents of the compound comprising a (C₄-C₅)alkylsulfonamide moiety, preferably 1 equivalent; the amount of iodine source is comprised from 10 mol% to 25 mol% of the moles of the compound comprising a (C₄-C₅)alkylsulfonamide moiety; preferably 15 mol% of the moles of the compound comprising a (C₄-C₅)alkylsulfonamide moiety; and the process is carried out in the presence of a mixture of acetonitrile/*tert*-butanol.

The process of the invention is carried out at a temperature comprised from 0°C to 50°C; preferably comprised from 20°C to 35°C; more preferably comprised from 20°C to 25°C. It is advantageous because the process can be carried out under mild conditions without compromizing the conversion into the 5- or 6- membered nitrogen-containing heterocyclic compound. Further, when the 5- or 6- membered nitrogen-containing heterocyclic compound can be in form of several diastereoisomers, the process of the invention mainly renders one of them as can be shown in the entries 38, 39 and 51 of Table 3.

The process of the invention is carried out under exposure to a source of light, particularly under exposure to a source of visible light. In an embodiment of the invention, optionally in combination with one or more features of the various embodiments described above or below, the process is that wherein the source of light is daylight. It is advantageous because in contrast to the processes of the state of the art, a light bulb is no longer required. The exposure of the reaction to a source of light is crucial for the reaction to progress. In the absence of light irradiation, the amination does not occur. However, admission of light leads to initiation of the reaction. In an embodiment of the invention, optionally in combination with one or more features of the various embodiments described above or below, the process is continuously under exposure to a source of light. The continuous exposure to a source of visible light allows increasing the yield by 1-2% by weight due to the continuing radical chain progress. In an embodiment, optionally in combination with one or more features of the various embodiments described above or below, the wavelength of the source of visible light is comprised from 350 nm to 820 nm; preferably from 350 nm to 450 nm; more preferably from 380 nm to 420 nm. In a preferred embodiment, optionally in combination with one or more features of the various embodiments described above or below, the wavelength of the source of visible light is 400 nm. In an embodiment, optionally in combination with one or more features of the various embodiments described above or below, the process of the invention is carried out under continuous exposure to a source of visible light having a wavelength comprised from 350 nm and 450 nm; preferably having a wavelength of 400 nm.

In an embodiment, optionally in combination with one or more features of the various embodiments described above or below, the process of the invention is carried out at a temperature comprised from 20 °C to 35 °C; and the source of light is daylight.

The second aspect of the invention refers to a compound of formula I(OCOR₁₁), wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group consisting of fluoro, chloro and bromo.

In an embodiment, optionally in combination with one or more features of the various embodiments described above or below, the compound is that of formula I(OCOR₁₁), wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group consisting of fluoro, chloro and bromo.

In an alternative embodiment, optionally in combination with one or more features of the various embodiments described above or below, the compound is that wherein R₁₁ is selected from the group consisting of (C₄-C₆)alkyl; preferably R₁₁ is -C(CH₃)₃, and the compound has the formula I(OCOC(CH₃)₃).

In an alternative embodiment, optionally in combination with one or more features of the various embodiments described above or below, the compound is that wherein R₁₁ is a phenyl substituted at any of the positions 2, 3, 5 and 6 with one or more radicals independently selected from halogen; preferably, R₁₁ is 3-chlorophenyl and the compound has the formula I(OCO-3-chlorophenyl).

In an embodiment, optionally in combination with one or more features of the various embodiments described above or below, the compound of formula I(OCOR₁₁) wherein R₁₁ is as defined above is obtainable by the process which comprises contacting one equivalent of an iodine source with one equivalent of a metal-free oxidant of formula PhI(OCOR₁₁); or, alternatively of formula R₁₁COOOH in the presence of an organic solvent; wherein the iodine source is selected from the group consisting of molecular iodine, N-iodosuccinimide, and salts of formula MI, wherein M is a cation selected from the group consisting of H⁺, a cation of an alkaline metal and a cation of a compound of formula N(R₁₂)₄, being R₁₂ selected from the group consisting of hydrogen and (C₁-C₆)alkyl; preferably the iodine source is selected from the group consisting of molecular iodine (I₂), Nal, N-iodosuccinimide, N(CH₃)₄I and KI; and even more preferably, the source of iodine is selected from the group consisting of molecular iodine and KI.

In an embodiment, optionally in combination with one or more features of the various embodiments described above or below, the compound of formula I(OCOR₁₁) wherein R₁₁ is phenyl as defined above is obtainable by the process which comprises contacting one equivalent of an iodine source with one equivalent of a metal-free oxidant of formula PhI(OCOR₁₁); or, alternatively of formula R₁₁COOOH in the presence of an organic solvent; wherein the iodine source is preferably selected from the group consisting of iodine and KI.

In an embodiment, optionally in combination with one or more features of the various embodiments described above or below, the compound of formula I(OCOR₁₁) wherein R₁₁ is (C₄-C₆)alkyl as defined above is obtainable by the process which comprises contacting one equivalent of an iodine source with one equivalent of a metal-free oxidant of formula PhI(OCOR₁₁) in the presence of an organic solvent; wherein the iodine source is preferably selected from the group consisting of iodine and KI.

It is also part of the invention a process for preparing the compound of formula I(OCOR₁₁) of the second aspect of the invention as defined above. This process comprises contacting an equivalent of the iodine source with an equivalent of corresponding metal-free oxidant in the presence of an organic solvent.

In an alternative embodiment, optionally in combination with one or more features of the various embodiments described above or below, wherein when in the compound of formula I(OCOR₁₁) R₁₁ is (C₄-C₆)alkyl; preferably when R₁₁ is -C(CH₃)₃, then the process comprises contacting an iodine source with a metal-free oxidant of formula PhI(OCO(C₄-C₆)alkyl)₂; preferably PhI(OCOC(CH₃)₃)₂ in the presence of an organic solvent.

In an alternative embodiment, optionally in combination with one or more features of the various embodiments described above or below, wherein when in the compound of formula I(OCOR₁₁) R₁₁ is a phenyl group as defined above the process comprises contacting an iodine source with a metal-free oxidant of formula PhI(OCOR₁₁)₂ being R₁₁ a phenyl group substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group consisting of fluoro, chloro and bromo; preferably PhI(OCO-3-chlorobenzoate)₂ in the presence of an organic solvent. Alternatively, the process comprises contacting an iodine source with a compound of formula R₁₁COOOH being R₁₁ a phenyl group substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group consisting of fluoro, chloro and bromo; preferably *meta*-Chloroperbenzoic acid (mCPBA).

In an embodiment of the invention, optionally in combination with one or more features of the various embodiments described above or below, the process for preparing the compound of formula I(OCOR₁₁) comprises preparing it "in situ". The term "in situ" refers to a process that is carried out in the reaction medium. In the context of the present invention, accordingly, the compound of formula I(OCOR₁₁) is prepared in the same enclosure where the conversion of the compound comprising a (C₄-C₅)alkylsulfonamide moiety into the 5- or 6-membered nitrogen-containing heterocyclic compound takes place by the catalytic process as defined in the presence invention. The preparation of the compound of formula I(OCOR₁₁) can be carried out in a previous step and/or during the progress of the process.

All the embodiments disclosed above for the iodine source and the metal-free oxidant defined above for the first aspect of the invention as well as the reaction conditions of the process for preparing a 5- or 6-membered nitrogen-containing heterocyclic compound applies also for the compound of formula I(OCOR₁₁) of the second aspect of the invention.

As shown in the Examples, the compound of the second aspect of the invention is useful as a catalyst for the preparation of a wide range of 5- or 6-membered nitrogen-containing heterocyclic compounds under mild conditions and under exposure to a source of light. In an embodiment, optionally in combination with one or more features of the various embodiments described above or below, the mixture of the iodine source and the metal-free oxidant as defined in the present invention, is that which is capable of being a catalyst under exposure to a source of visible light.

The third aspect of the invention refers to the use of the compound as defined in the second aspect of the invention as a catalyst wherein the process is carried out under exposure to a source of light; particularly to a source of visible light. In a particular embodiment, the compound of the invention is useful for the preparation of 5- or 6-membered nitrogen-containing heterocyclic compounds; particularly to pyrrolidines and derivated pyrrolidine structures, as described in the first aspect of the invention.

All the embodiments disclosed above for the process of the first aspect of the invention as well as for the compound of the second aspect of the invention applies also for the use of the compound as catalyst of the third aspect of the invention.

The fourth aspect of the invention refers to a compound of formula I[(OCOR₁₁)₂]M, wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group fluoro, chloro and bromo; and M is a cation selected from the group consisting of H⁺, a cation of an alkaline metal and a cation of a compound of formula N(R₁₂)₄] wherein each R₁₂ is independently selected from the group consisting of hydrogen and (C₁-C₆)alkyl. These compounds are synthethic intermediates for the preparation of the compound of formula I(OCOR₁₁) as defined in the second aspect of the invention. As it is shown above in the scheme of the process of the invention, the intermediate compound of formula I[(OCOR₁₁)₂]M can be tranformed in the active specie of formula I(OCOR₁₁).

In an embodiment of the invention, optionally in combination with one or more features of the various embodiments described above or below, a compound of formula I[(OCOR₁₁)₂]M is obtainable by contacting an iodine source as defined above with a metal-free oxidant as defined in the present invention. It is also part of the invention a process for preparing the compound of formula I[(OCOR₁₁)₂]M as defined in the fourth aspect of the invention. This process comprises contacting the iodine source with the corresponding metal-free oxidant in the presence of an organic solvent. In a particular embodiment, optionally in combination with one or more features of the various embodiments described above or below, the process for preparing a compound of formula I[(OCOR₁₁)₂]M as defined above comprises reacting an equivalent of the iodine source MI with an equivalent of a metal-free oxdiant of formula I(OCOR₁₁)₂ being M and R₁₁ as defined in the present invention

In an embodiment of the invention, optionally in combination with one or more features of the various embodiments described above or below, the compound of formula I[(OCOR₁₁)₂]M is that wherein M is H⁺.

In an embodiment of the invention, optionally in combination with one or more features of the various embodiments described above or below, the compound of formula I[(OCOR₁₁)₂]M is that wherein M is a cation of an alkaline metal selected from the group consisting of sodium and potassium; preferably potassium.

In an alternative embodiment of the invention, optionally in combination with one or more features of the various embodiments described above or below, the compound of formula I[(OCOR₁₁)₂]M is that wherein M is a cation of a compound of formula N(R₁₂)₄] wherein each R₁₂ is independently selected from hydrogen, methyl, ethyl and butyl.

All the embodiments disclosed above for the compound of formula I(OCOR₁₁) of the second aspect of the invention can applies also for the compound of formula I[(OCOR₁₁)₂]M of the fourth aspect of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

All solvents, reagents and all deuterated solvents were purchased from Aldrich and TCI commercial suppliers. Column chromatography was performed with silica gel (Merck, type 60, 0.063-0.2mm). NMR spectra were recorded on a Bruker Avance 400 MHz or 500 MHz spectrometer, respectively. All chemical shifts in NMR experiments were reported as ppm downfield from TMS. The following calibrations were used: CDCl₃ d = 7.26 and 77.0 ppm, CD₂Cl₂ d = 5.32 and 54.00 ppm, (CD₂Cl)₂d = 5.32. MS (ESI-LCMS) experiments were performed using an Agilent 1100 HPLC with a Bruker micro-TOF instrument (ESI). A Supelco C8 (5 cm x 4.6 mm, 5 µm particles) column was used with a linear elution gradient from 100% H₂O (0.5% HCO₂H) to 100% MeCN in 13 min at a flow rate of 0.5 mL/min. MS (EI) and HRMS experiments were performed on a Kratos MS 50. IR spectra were taken in a Bruker Alpha instrument in the solid state.

The following compounds were commercially available and used as received: isobutyronitrile, diphenylacetonitrile, cyclohexanecarbonitrile, a-methylbenzylcyanide, (2-bromoethyl)benzene, 1-bromo-3-phenylpropane, 4-fluorophenethyl bromide, 4-chlorophenethyl bromide, 4-bromophenethyl bromide, 4-methylphenethyl bromide, 4-methoxyphenethyl bromide, 1-bromobutane, 1-bromo-2-methylpropane, 1-bromoetane, 2-bromoethyl methyl ether, trans-2-bromo-1-indanol, 2-(1,2,3,4-tetrahydronaphthalen-2yl)ethan-1-amine, N-(2-aminoethyl)-N-benzylmethanesulfonamide, 4-phenylbutylamine, p-toluenesulfonyl chloride, methanesulfonyl chloride, 4-nitrobenzenesulfonyl chloride, 2-(trimethylsilyl)ethanesulfonyl chloride, benzyl chloride, 3-chlorobenzoic acid (meta-chlorobenzoic acid; mCBA), 3-chloroperbenzoic acid (*meta*-Chloroperbenzoic acid; mCPBA), (diacetoxyiodo)benzene, peracetic acid solution, *tert*-butyl hydroperoxide solution, hydrogen peroxide solution, iodine, trifluoroacetic acid, pivalic acid, tetrabutylammonium iodide, potassium iodide and sodium hypochloride solution. lodobenzene dipivalate and (di-m-chlorobenzoyldioxyiodo)benzene were synthesised according to procedures described in the state of the art (cf. J. A. Souto, C. Martínez, I. Velilla, K. Muñiz, " Defined Hypervalent Iodine(III) Reagents Incorporating Transferable Nitrogen Groups: Nucleophilic Amination through Electrophilic Activation", Angew. Chem. Int. Ed. 2013, vol. 52, pages: 1324-1328; and B. Plesnicar, G. A. Russell, "Synthesis and Reactions of Substituted (Dibenzoyldioxyiodo)benzenes; A New Class of Organic Oxidants", Angew. Chem. Int. Ed. 1970, vol. 9, pages 797-798).

### 1. General procedure for the synthesis of compounds comprising a (C₄-C₅)alkylsulfonamide moiety (compounds of formula (1))

Compounds comprising a (C₄-C₅)alkylsulfonamide moiety were synthesized following a three-step procedure as outlined below:

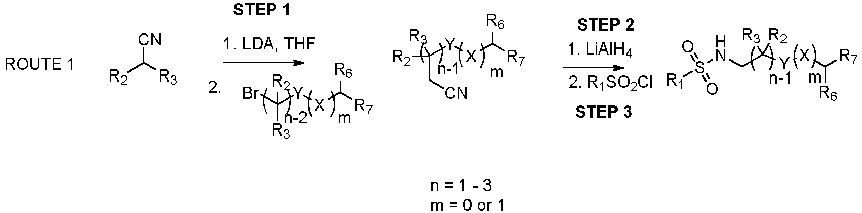

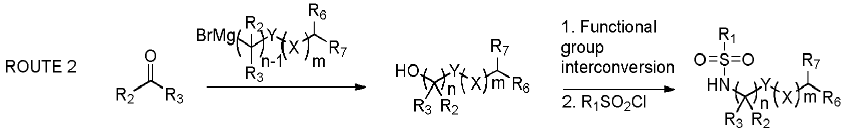

### Step 1

A Schlenk tube equipped with a stirrer bar was charged with the corresponding nitrile compound (1.0 equiv.) and THF (tetrahydrofurane; 50 mL). LDA (lithium diisopropylamide; 2.6 mL, 2M, 1.0 equiv.) was added dropwise at -78 °C and the solution was stirred for 30 min. After that period, the corresponding bromide (1.2 equiv.) was added in a single portion and the mixture was stirred at room temperature for 12h. A saturated aqueous solution of NH₄Cl was added and the resulting mixture was extracted with CH₂Cl₂ (3x). The organic layer was dried over sodium sulfate (Na₂SO₄) and the solvent was evaporated under reduced pressure. The crude product was directly used for the subsequent reduction step.

### Step 2

A flame dried Schlenk equipped with a stirrer bar and a reflux condenser was charged with lithium aluminium hydride LiAlH₄ (3 equiv.), diethyl ether (Et₂O) was added carefully and the mixture was cooled to 0 °C with an external ice/water cooling bath. The crude nitrile (1 equiv.) was dissolved in a small volume of Et₂O and added carefully to the LiAlH₄ suspension. The mixture was heated to reflux for 2h and cooled to 0 °C afterwards. A solution of NaOH (10% in water) was added carefully until a white solid precipitates. After filtration over MgSO₄ and evaporation of the solvent the crude amine was obtained in quantitative yields.

### Step 3

The crude amine from step 2 (1 equiv.) was dissolved in pyridine (50 mL) and the respective sulfonyl chloride (1.5 equiv.) was added at 0 °C. The solution was stirred overnight at 25 °C. CH₂Cl₂ was added, and the mixture was washed three times with a hydrochloride solution (10% HCl in water). The organic layer was dried over Na₂SO₄ a the solvent was evaporated under reduced pressure. The crude product was purified by chromatography (silica gel, n-hexane/ethyl acetate, 4/1, v/v) to give the pure compound comprising a (C₄-C₅)alkylsulfonamide moiety of formula (1).

### 2. General procedure for C-H amination reactions (compounds of formula (2))

A Schlenk tube equipped with a stirrer bar was charged with a metal-free oxidant, I₂ (iodine source) and a compound comprising a (C₄-C₅)alkylsulfonamide moiety of formula 1, evacuated, and backfilled with argon, before 1.5 mL of the appropriate solvent was added. The solution was stirred at 25 °C for the appropriate period of time under exposure to daylight CH₂Cl₂ was added and the resulting solution was washed with saturated aqueous solutions of Na₂SO₃ and NaHCO₃ and extracted with CH₂Cl₂ (3x). The combined organic layers were dried over Na₂SO₄ and solvents were evaporated under reduced pressure. The crude aminated 5- or 6-membered ring compound was purified by chromatography (silica gel, n-hexane/ethyl acetate, 4/1, v/v) to give the pure 5- or 6-membered nitrogen-containing heterocyclic compound of formula (2).

The type and amount of metal-free oxidant, and compound comprising a (C₄-C₅)alkylsulfonamide moiety of formula (1) used in the process of the invention, as well as the amount of I₂ and the reaction conditions are disclosed in Tables 1, 2 and 3.

Processes disclosed in Tables 1 and 2 are summarized in Scheme 1:

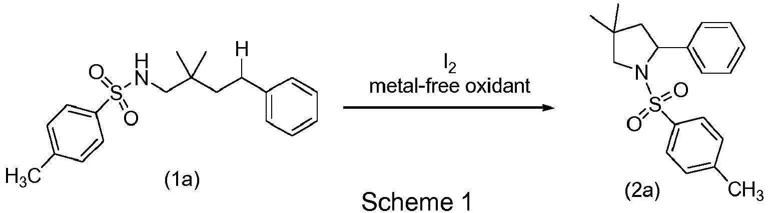

Processes for C-H amination reactions disclosed in Table 1 were carried out in the presence of dichloroethane as a solvent, and the reaction mixture was stirred at 25°C for 12h under continuous exposure to a source of visible light.

**Table 1**

| **Entry** | **I₂** (**mol**%) | **Metal-free oxidant** | | **2a Conversion (%)** |
|---|---|---|---|---|
| | | **Type** | **Amount (eq.)** | |
| 1 | 20 | PhI(O₂C(CH₃)₃)₂ | 3 | 79 |
| 2 | 20 | PhI(*m*CBA)₂^{a} | 3 | 98 |
| 3 | 20 | PhI(*m*CBA)₂^{a} | 1 | 98 |
| 4 | 5 | PhI(*m*CBA)₂^{a} | 1 | 98 |
| 5 | 2.5 | PhI(*m*CBA)₂^{a} | 1 | 98 |
| 6 | 1 | PhI(*m*CBA)₂^{a} | 1 | 95 |
| 7 | 0.5 | PhI(*m*CBA)₂^{a} | 1 | 76 |
| 8^{b} | 0.5 | PhI(*m*CBA)₂^{a} | 1 | 85 |
| 9^{c} | 5 | PhI(*m*CBA)₂^{a} | 1 | 98 |
| 10^{d} | 2.5 | PhI(*m*CBA)₂^{a} | 1 | 98 |

| | | | | |
|---|---|---|---|---|
| ^{a} *m*CBA refers to 3-chlorobenzoic acid. The metal-free oxidant is PhI(O₂C-3-chloro(Ph))₂. ^{b} the reaction was carried out at 400 nm wavelength irradiation. ^{c} the reaction was carried out on 13 mmol scale. ^{d} the reaction was carried out in the presence of 10 equivalents of *m*CBAH. | | | | |

Processes for C-H amination reactions disclosed in Table 2 were carried out in the presence of MCPBA (meta-chloroperbenzoic acid) as a metal-free oxidant, and the reaction mixture was stirred at 25°C under exposure to a source of visible light for the period of time specified in Table 2.

**Table 2**

| **Entry** | **I₂ (mol%)** | **MCPBA (eq.)** | **Solvent** | **Time (h)** | **2a Conversion (%)** |
|---|---|---|---|---|---|
| 11 | 15 | 3 | CH₃CN/CH₃COOH (1:1) | 12 | 52^{c} |
| 12 | 15 | 1 | CH₃CN | 12 | 44^{c} |
| 13 | 15 | 1 | CH₃CN | 24 | 51^{c} |
| 14 | 15 | 1 | CH₃CN | 48 | 60^{c} |
| 15 | 15 | 1^{a} | CH₃CN | 24 | 40^{c} |
| 16 | 15 | 3 | PhCN | 12 | 38^{c} |
| 17 | 15 | 3 | EtOAc | 12 | 56^{c} |
| 18 | 15 | 3 | CH₃CN | 12 | 60^{c} |
| 19 | 15 | 1 | CH₃CN/^{t}BuOH (1:1) | 12 | 98^{b} |
| 20 | 10 | 1 | CH₃CN/^{t}BuOH (1:1) | 12 | 85^{b} |
| 21 | 5 | 1 | CH₃CN/^{t}BuOH (1:1) | 12 | 70^{b} |
| 22 | 2.5 | 1 | CH₃CN/^{t}BuOH (1:1) | 12 | 56^{b} |
| 23 | 2.5 | 3 | CH₃CN/^{t}BuOH (1:1) | 24 | 60^{b} |

| | | | | | |
|---|---|---|---|---|---|
| ^{a} The amount of MCPBA was added in two portions. ^{b} Isolated yield after purification. ^{c} Estimated yield from integration in the crude ¹H-NMR spectrum. | | | | | |

Processes for C-H amination reactions disclosed in Table 3 were carried out in the presence of 2.5 mol% of I₂ as a iodine source, 1.0 equiv. of PhI(*m*CBA)₂ as a metal-free oxidant, and the reaction mixture was carried out in the presence of (CH₂Cl)₂ and under stirring at 25°C under continuous exposure to a source of visible light for 12h. The compound comprising a (C₄-C₅)alkylsulfonamide moiety of formula (1), 5- or 6-membered nitrogen-containing heterocyclic compound of formula (2), and the yield of conversion are disclosed in Table 3.

**Table 3**

| **Entry** | **Compound 1** | **Compound 2** | **Conversion^{a}** | |
|---|---|---|---|---|
| | | | **Yield (%)** | **d iastereoselectivity** |
| 24 | 1b | 2b | 63 | - |
| 25 | 1c | 2c | 88 | - |
| 26 | 1d | 2d | 78 | - |
| 27 | 1e | 2e | 78 | - |
| 28 | 1f | 2f | 99 | - |
| 29 | 1g | 2g | 83 | - |
| 30 | 1h | 2h | 82 | - |
| 31 | 1i | 2i | 73 | - |
| 32 | 1j | 2j | 96 | - |
| 33 | 1k | 2k | 99 | - |
| 34 | 1l | 2l | 58 | - |
| 35 | 1m | 2m | 80 | - |
| 36 | 1n | 2n | 90 | - |
| 37 | 1o/1o' | 2o/2o' | 97 | 1:1 |
| 38 | 1p | 2p | 99 | 100:0 |
| 39 | 1q | 2q | 83 | 100:0 |
| 40 | 1r | 2r | 75 | - |
| 41 | 1s | 2s | 84 | - |
| 42 | 1t | 2t | 65 | - |
| 43 | 1u | 2u | 79 | - |
| 44 | 1v | 2v | 79 | - |
| 45 | 1w | 2w | 80 | - |
| 46 | 1x | 2x | 81 | - |
| 47 | 1y | 2y | 95 | - |
| 48 | 1z | 2z | 91 | - |
| 49 | 1aa | 2aa | 84 | - |
| 50 | 1ab^{b} | 2ab | 72 | - |
| 51 | 1ac^{b} | 2ac | 82 | 100:0 |
| 52 | 1ad | 2ad | 90 | - |
| 53 | 1ae | 2ae | 77 | - |

| | | | | |
|---|---|---|---|---|
| ^{a} Isolated yield after chromatographic purification. ^{b} The amount of I₂ was 10mol%. | | | | |

The results of Tables 1-3 show that the process of the invention allows preparing a wide range of 5- or 6-membered nitrogen-containing heterocyclic compound under mild conditions, in the absence of metal catalyst and without compromising the conversion and diastereoselectivity of the reaction. Further, the process of the invention is an operatioanlly simple process that proceeds with a catalytically amount of iodine source (lower than the stoichiometric amount), and even at stoichiometric amount of the metal-free oxidant.

Mass spectrometry data for the compounds of formula 2a-2ae are summarized in Table 4.

**Table 4**

| Compound | High Resolution Mass (g/mol) | |
|---|---|---|
| | Calculated | Found |
| 2a | 352.1242 | 352.1352 |
| 2b | 383.1036 | 383.1037 |
| 2c | 362.1580 | 362.1578 |
| 2d | 476.1651 | 476.1655 |
| 2e | 378.1522 | 378.1513 |
| 2f | 392.1655 | 392.1661 |
| 2g | 348.1428 | 348.1431 |
| 2h | 386.0952 | 386.0952 |
| 2i | 430.0446 | 430.0448 |
| 2j | 366.1498 | 366.1490 |
| 2k | 360.1628 | 360.1636 |
| 2l | 452.1655 | 452.1647 |
| 2m | 336.1029 | 336.1027 |
| 2n | - | 324[M+Na]⁺/302 [M+H]⁺ |
| 2o/2o' | 316.1366 | 316.1367 |
| 2p | 336.1035 | 336.1029 |
| 2q | 350.1185 | 350.1189 |
| 2r | 338.1185 | 338.1172 |
| 2s | 400.1342 | 400.1344 |
| 2t | 344.1679 | 344.1674 |
| 2u | 406.1835 | 406.1839 |
| 2v | 304.1342 | 304.1353 |
| 2w | 276.1029 | 276.1029 |
| 2x | 304.1342 | 304.1327 |
| 2y | 306.1134 | 306.1136 |
| 2z | 303.0929 | 303.0930 |
| 2aa | 403.0765 | 403.0757 |
| 2ab | 340.0978 | 340.0979 |
| 2ac | 414.1134 | 414.1131 |
| 2ad | 232.0403 | 232.0414 |
| 2ae | 505.1226 | 505.1230 |

### REFERENCES CITED IN THE APPLICATION

- Renhua Fan et al., "δ and α SP3 C-H bond oxidation of sulfonamides with PhI(OAc)2/I2 under Metal-free conditions", J.Org. Chem., 2007, vol. 72, pp. 8994-8997
- J. A. Souto, C. Martínez, I. Velilla, K. Muñiz, " Defined Hypervalent Iodine(III) Reagents Incorporating Transferable Nitrogen Groups: Nucleophilic Amination through Electrophilic Activation", Angew. Chem. Int. Ed. 2013, vol. 52, pages 1324-1328
- B. Plesnicar, G. A. Russell, "Synthesis and Reactions of Substituted (Dibenzoyldioxyiodo)benzenes; A New Class of Organic Oxidants", Angew. Chem. Int. Ed. 1970, vol. 9, pages 797-798).

## Claims

1. A process for preparing a 5- or 6-membered nitrogen-containing heterocyclic compound which comprises contacting a compound comprising a (C₄-C₅)alkylsulfonamide moiety, with a source of iodine and a metal-free oxidant;
wherein:
the compound comprising a (C₄-C₅)alkylsulfonamide moiety is capable of suffering an intramolecular cyclization converting a C-H bond into a C-N bond, in order to form a 5- or 6-membered nitrogen-containing heterocyclic ring;
the amount of the iodine source is comprised from 0.5 mol% to 50 mol% of the moles of the compound comprising a (C₄-C₅)alkylsulfonamide moiety; and
the amount of metal-free oxidant is comprised from 1 to 5 equivalents of the compound comprising a (C₄-C₅)alkylsulfonamide moiety; and
the process is carried out at a temperature comprised from 0°C to 50°C in the presence of an organic solvent and under exposure to a source of light.

2. The process according to claim 1, wherein the compound comprising a (C₄-C₅)alkylsulfonamide moiety is a compound of formula (1), and the 5- or 6-membered nitrogen-containing heterocyclic compound is a compound of formula (2): 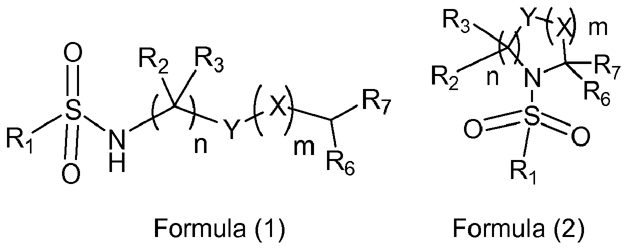 wherein:
X is a heteroatom selected from the group consisting of NPG, O and S;
Y is selected from the group consisting of CR₄R₅ and C=CR₄R₅;
R₁ is selected from (C₁-C₅)alkyl and phenyl, being the groups alkyl and phenyl unsubstituted or substituted at any position with one or more radicals independently selected from the group consisting of (C₁-C₅)alkyl, nitro, ((C₁-C₄)alkyl)₃-Si-, halogen and hydroxy;
R₂, R₃, R₄, R₅, R₆ and R₇ are independently selected from the group consisting of hydrogen, (C₁-C₆)alkyl, monocyclic ring, benzyl, (C₂-C₆)alkenyl, (C₂-C₆)alkynyl, being the groups (C₁-C₆)alkyl, benzyl, (C₂-C₆)alkenyl and (C₂-C₆)alkynyl unsubstituted or substituted at any position with one or more radicals independently selected from the group consisting of halogen, hydroxy, N(R₁₂)₂ wherein each R₁₂ is independently selected from hydrogen and (C₁-C₆)alkyl, (C₁-C₆)alkyl-O-; or alternatively,
R₂ and R₃; R₄ and R₅; R₆ and R₇; R₂ and R₄; R₂ and R₅; R₃ and R₄; and R₃ and R₅; in combination with the adjacent carbon atoms form a 5 to 6 membered ring, saturated or partially unsaturated, optionally bridged or fused to a 5 to 6 membered ring; the members of the rings being independently selected from C, CH, CH₂, O, N, NH, and S; being one or more of the hydrogen atoms of the members optionally substituted by a radical selected from the group consisting of (C₁-C₅)alkyl, nitro, halogen and hydroxy;
PG is an amine protecting group selected from the group consisting of the groups of formula -(CO)OR₈; benzyl, and -SO₂R₉;
R₈ is selected from the group consisting of (C₁-C₆)alkyl, benzyl and fluorenyl;
R₉ is selected from (C₁-C₅)alkyl and phenyl, being the groups alkyl and phenyl unsubstituted or substituted at any position with one or more radicals independently selected from the group consisting of (C₁-C₅)alkyl, nitro, ((C₁-C₄)alkyl)₃-Si-, halogen and hydroxy;
n is an integer selected from 1-3;
m is an integer selected from 0-1; and
with the proviso that when m is 0 then n is 2 or 3, and when m is 1, then n is 1 or 2.

3. The process according to any of the claims 1-2, wherein the source of iodine is selected from the group consisting of molecular iodine and KI.

4. The process according to any of the claims 1-3, wherein the metal-free oxidant is selected from the group consisting of bleach, a peroxide, a peracid, an hypervalent iodine(III) reagent, and a persulfate.

5. The process according to any of the claims 1-4, wherein:
(a) the metal-free oxidant is a hypervalent iodine(III) reagent of formula PhI(Z)₂, wherein Z is selected from -OCO(C₁-C₆)alkyl and -OCOR₁₀; and R₁₀ is phenyl; being the groups alkyl or phenyl unsubstituted or substituted at any position with one or more radicals independently selected from the group consisting of (C₁-C₅)alkyl, (C₁-C₅)alkyl-O-,halogen and hydroxy; or alternatively
(b) the metal-free oxidant is a peracid selected from the group consisting of 3-chloroperbenzoic acid and peracetic acid.

6. The process according to claim 5, wherein the metal-free oxidant is a hypervalent iodine(III) reagent of formula PhI(OCOC(CH₃)₃)₂ or Phl(3-chlorobenzoate)₂; or alternatively the metal-free oxidant is 3-chloroperbenzoic acid.

7. The process according to any of the claims 1-6, wherein:
(a) when the metal-free oxidant is a hypervalent iodine(III) reagent of formula PhI(OCO(C1C₆)alkyl)₂, then the amount of iodine source is comprised from 20 mol% to 50 mol% of the moles of the compound comprising a (C₄-C₅)alkylsulfonamide moiety; or alternatively
(b) when the metal-free oxidant is a hypervalent iodine(III) reagent of formula PhI(OCOR₁₀)₂, then the amount of iodine source is comprised from 0.5 mol% to 5 mol% of the moles of the compound comprising a (C₄-C₅)alkylsulfonamide moiety; or alternatively
(c) when the metal-free oxidant is a peracid, then the amount of iodine source is comprised from 2 mol% to 25 mol% of the moles of the compound comprising a (C₄-C₅)alkylsulfonamide moiety.

8. The process according to any of the claims 1-7, wherein the amount of metal-free oxidant is comprised from 1 to 3 equivalents of the compound comprising a (C₄-C₅)alkylsulfonamide moiety.

9. The process according to any of the claims 1-8, wherein:
(a) when the metal-free oxidant is a hypervalent iodine(III) reagent, then the solvent is a chlorinated solvent; or alternatively
(b) when the metal-free oxidant is a peracid, then the solvent is acetonitrile/*tert*-butanol.

10. The process according to any of the claims 1-9, wherein the temperature is comprised from 20°C to 35°C; and the source of light is daylight.

11. A compound of formula I(OCOR₁₁), wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group consisting of fluoro, chloro and bromo.

12. The compound according to claim 11, having the formula I(OCOC(CH₃)₃) or alternatively the formula I(OCO-3-chlorophenyl).

13. The compound according to any of the claims 11-12, obtainable by the process which comprises contacting one equivalent of an iodine source selected from the group consisting of molecular iodine and KI, with one equivalent of a metal-free oxidant of formula PhI(OCOR₁₁); or, alternatively of formula R₁₁COOOH in the presence of an organic solvent.

14. Use of the compound as defined in any of the claims 11-13, as a catalyst to be used under exposure to a source of light.

15. A compound of formula I[(OCOR₁₁)₂]M, wherein R₁₁ is selected from the group consisting of (C₄-C₆)alkyl and phenyl substituted at any of the positions 2, 3, 5 and 6 with one or more radicals independently selected from the group consisting of (C₁-C₅)alkyl, (C₁-C₅)alkyl-O-, hydroxy and halogen, being the halogen group selected from the group fluoro, chloro and bromo; and M is a cation selected from the group consisting of H⁺, a cation of an alkaline metal and a cation of a compound of formula N(R₁₂)₄] wherein each R₁₂ is independently selected from the group consisting of hydrogen and (C₁-C₆)alkyl.
